# EUROPEAN PATENT APPLICATION

(11) **EP 4 748 298 A1**
(43) Date of publication of application: **27.05.2026**
(21) Application number: 25213666.8
(22) Date of filing: 05.11.2025
(51) Int. Cl.: A61B 5/00, A61B 5/01, A61B 5/1495

(54) **WEARABLE COMPUTING DEVICE WITH TEMPERATURE CORRECTION OF SKIN AUTOFLUORESCENCE SIGNAL**

(30) Priority: 26.11.2024 US 202418961222
(71) Applicant: GOOGLE LLC, Mountain View CA 94043 (US)
(72) Inventor: Watkins, Herschel Max, Mountain View, 94043 (US); Yao, Kaiyuan, Mountain View, 94043 (US); Anderson-Conway, Louise Maud Alice, Mountain View, 94043 (US); Vavadi, Hamed, Mountain View, 94043 (US)
(74) Representative: Maikowski & Ninnemann Patentanwälte Partnerschaft mbB

(57) **Abstract**

A computing device includes a temperature sensor for generating skin temperature data of a user of the computing device, a skin autofluorescence (SAF) sensor for generating SAF data of the user, and a controller configured to perform a plurality of operations. The plurality of operations includes receiving the skin temperature data and the SAF data over time, determining a correlation between the skin temperature data and the SAF data over time, applying a temperature correction factor to the SAF data to correct the SAF data for skin temperature of the user based on the correlation, determining at least one health parameter of the user using the corrected SAF data, and displaying, via a display of the computing device, the at least one health parameter to the user.

## Description

### FIELD OF THE INVENTION

The present disclosure relates generally to wearable computing devices, and more particularly, to a wearable computing device having temperature correction of a skin autofluorescence signal to improve biometric sensing.

### BACKGROUND

Recent advances in technology, including those available through consumer devices, have provided corresponding advances in health detection and monitoring. For instance, wearable computing devices such as fitness trackers and smart watches, include a variety of sensors for measuring multiple biological parameters and, optionally, for enabling other features, such as GPS guidance, music, communications, payments, and/or the like that can be beneficial to a user of the device.

Skin autofluorescence (SAF) can be used to non-invasively detect the presence of and measure levels of advanced glycation end products (AGEs) that are present below the surface of a person's skin. AGEs are biomarkers that have been associated with aging and cardiovascular health and have also been implicated in such conditions as diabetes, atherosclerosis, kidney disease, and Alzheimer's disease. In particular, SAF can be strongly correlated with health outcomes and can be used in conjunction with other physiologic data (e.g., heart rate and oxygen saturation (SpO2)) to provide information to a person about the person's health.

Typically, a light source or emitter (e.g., a light emitting diode) and a light detector (e.g., a photodiode) are utilized in the devices that measure the intensity of returned light signals that are used to determine a level of SAF. Maximizing the value of SAF requires monitoring small changes in SAF over time. Unfortunately, SAF is sensitive to skin temperature, with higher temperatures resulting in lower SAF and vice versa. The magnitude of SAF change with moderate changes in skin temperature is large relative to the change in SAF expected from behavior change over a certain time period, such as a few months. This means that it can be difficult to interpret changes in SAF correctly.

As such, a need exists for a device, system, and method of monitoring skin temperature and minimizing or eliminating the interference of the skin temperature on SAF signals.

### SUMMARY OF THE INVENTION

Aspects and advantages of embodiments of the present disclosure will be set forth in part in the following description, or can be learned from the description, or can be learned through practice of the embodiments.

In an aspect, the present disclosure is directed to a computing device. The computing device includes a temperature sensor for generating skin temperature data of a user of the computing device, a skin autofluorescence (SAF) sensor for generating SAF data of the user, and a controller configured to perform a plurality of operations. The plurality of operations includes receiving the skin temperature data and the SAF data over time, determining a correlation between the skin temperature data and the SAF data over time, applying a temperature correction factor to the SAF data to correct the SAF data for skin temperature of the user based on the correlation, determining at least one health parameter of the user using the corrected SAF data, and displaying, via a display of the computing device, the at least one health parameter to the user.

In another aspect, the present disclosure is directed to a method of correcting SAF data based on skin temperature of a user. The method includes receiving, via a computing device, skin temperature data and the SAF data of the user over time. The method also includes determining, via the computing device, a correlation between the skin temperature data and the SAF data over time. Further, the method includes applying, via the computing device, a temperature correction factor to the SAF data to correct the SAF data based on the skin temperature of the user based on the correlation. Moreover, the method includes determining, via the computing device, at least one health parameter of the user using the corrected SAF data. In addition, the method includes displaying, via a display of the computing device, the health parameter(s) to the user.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1A illustrates a perspective view of a wearable computing device in accordance with aspects of the present disclosure, particularly with the wearable computing device being worn on a wrist of a user.
FIG. 1B illustrates a side view of the wearable computing device of FIG. 1A in accordance with aspects of the present disclosure.
FIG. 2 illustrates a schematic diagram of an example system that can be utilized with the wearable computing device in accordance with aspects of the present disclosure.
FIG. 3 illustrates a graph of advanced glycation end products (AGEs) data versus the skin temperature data in accordance with aspects of the present disclosure.

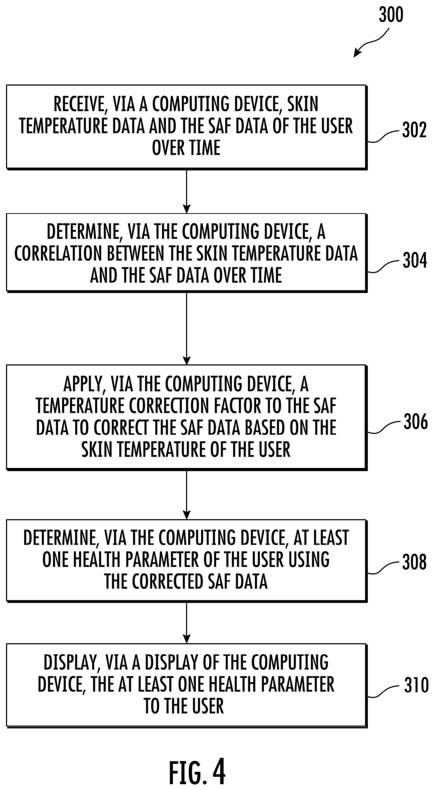

FIG. 4 illustrates a flow diagram of a method of correcting skin autofluorescence (SAF) data based on skin temperature of a user in accordance with aspects of the present disclosure.

Repeat use of reference characters in the present specification and drawings is intended to represent the same or analogous features or elements of the present technology.

### DETAILED DESCRIPTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope of the invention. For instance, features illustrated or described as part of one embodiment can be used with another embodiment to yield a still further embodiment. Thus, it is intended that the present invention covers such modifications and variations as come within the scope of the appended claims and their equivalents.

Generally, the present disclosure relates to a device, system, and method of correcting for skin temperature when evaluating changes in SAF signals over time. The device includes a temperature sensor for generating skin temperature data of a user of the computing device. The device also includes a SAF sensor for generating skin autofluorescence (SAF) data of the user. Furthermore, the system includes a controller configured to perform a plurality of operations, such as receiving the skin temperature data and the SAF data over time and determining a correlation between the skin temperature data and the SAF data over time. Moreover, the controller is configured to generate a temperature correction factor based on the correlation and apply the temperature correction factor to the SAF data to correct the SAF data for skin temperature of the user. In addition, the controller is configured to determine at least one health parameter of the user using the corrected SAF data and displaying, via a display of the computing device, the health parameter(s) to the user.

With reference now to the Figures, example embodiments of the present disclosure will be discussed in further detail.

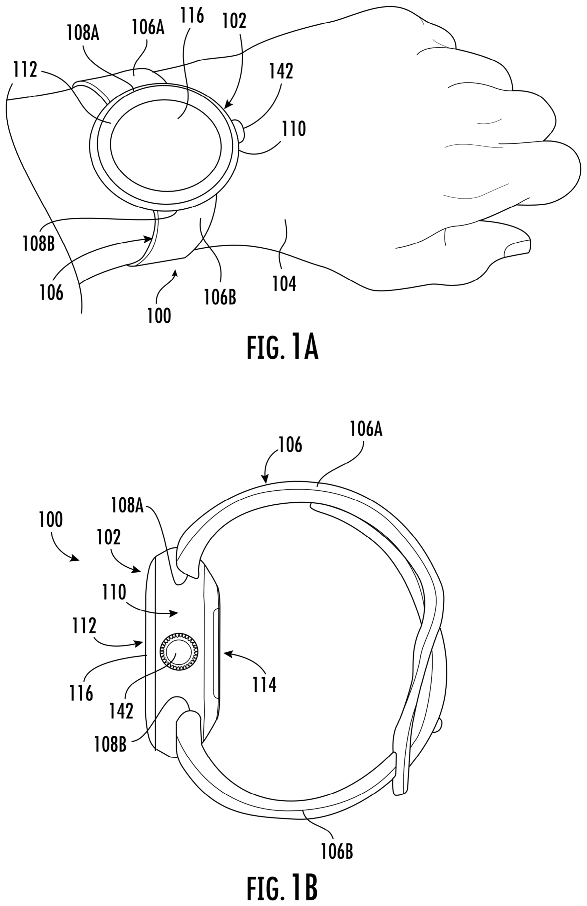

Referring now to the drawings, FIGS. 1A-1B illustrate various views of a wearable computing device 100 and components thereof according to the present disclosure. For instance, FIG. 1A illustrates a perspective view of the wearable computing device 100, particularly with the wearable computing device 100 being worn on the wrist of a user. FIG. 1B illustrates a side view of the wearable computing device 100 of FIG. 1A.

As generally shown in FIGS. 1A-1B, and as will be described below in greater detail, the wearable computing device 100 includes a housing 102 that contains electronics associated with the wearable computing device 100. In some instances, as shown in FIG. 1A, the wearable computing device 100 may be worn on a user's forearm 104 like a wristwatch. Thus, as shown, the wearable computing device 100 may include a wearable band, such as a wristband 106 having one or more parts, such as the two wristband straps 106A, 106B, for securing the wearable computing device 100 to the user's forearm 104. In such instance, the housing 102 may include one or more connection points (e.g., connection points 108A, 108B) at which the wristband 106 is couplable to the housing 102. For instance, the wristband 106 is shown coupled to the housing 102 at two locations (e.g., connection points 108A, 108B). However, it should be appreciated that the wearable computing device 100 may be worn at any other suitable location by a user, such as, for example, on an ankle, a torso, and/or the like.

In addition, as best shown in FIG. 1B, in some instances, the housing 102 has a main housing 110, an upper housing cover 112, and a rear housing cover 114. The main housing 110 of the housing 102 of the wearable computing device 100 generally ext. In some instances, the connection points 108A, 108B may be provided on the main housing 110. The main housing 110, the upper housing cover 112, and the rear housing cover 114 together generally define an exterior of the wearable computing device 100. Particularly, the upper housing cover 112 generally defines an upper surface of the wearable computing device 100, the rear housing cover 114 generally defines a rear surface of the wearable computing device 100, and the main housing 110 generally defines a side surface defined between the upper surface and the rear surface.

The upper and rear housing covers 112, 114 are connectable to the main housing 110 to at least partially enclose an interior volume of the wearable computing device 100. The interior volume of the wearable computing device 100 is configured to at least partially receive one or more components. For instance, as shown in FIG. 1A, an electronic display screen 116 may be received within the interior volume and viewable through at least the upper housing cover 112. For such purpose, in an embodiment, the upper housing cover 112 may be constructed of glass, polycarbonate, acrylic, or similar, or the electronic display screen 116 may form part of the upper housing cover 112. Moreover, in an embodiment, the electronic display screen 116 may cover an electronics package (not shown), which may also be housed within the housing 102. The electronic display screen 116 may be any suitable display type, such as a touch screen, organic light emitting diode (OLED), liquid crystal display (LCD), and/or the like. The rear housing cover 114 of the housing 102 may be configured to be closest to a user when worn. For instance, the rear housing cover 114 may contact a dorsal wrist of a user when being worn by the user, as shown in FIG. 1A.

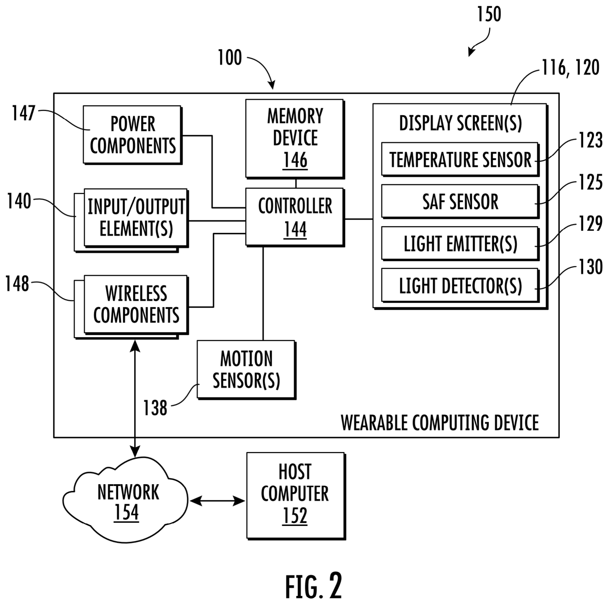

Additionally, in accordance with aspects of the present disclosure, the wearable computing device 100 may also include one or more sensors, such as optical sensors (such as photoplethysmography sensors or SAF sensors), temperature sensors (such as an ambient temperature sensor or a skin temperature sensor), a humidity sensor, a pressure sensor, one or more microphones, one or more biometric sensors, and/or the like. For instance, as shown in FIG. 2, the optical sensor(s) of the wearable computing device 100 may include one or more light emitters 129 and light detectors 130. Generally, the light detector(s) 130 may be used alone to detect ambient light (light not emitted from the wearable computing device 100) or may be used in combination with the light emitter(s) 129 such that the light detector(s) 130 detects both ambient light and at least a portion of the light emitted by the light emitter(s) 129 and reflected off a user or a surface and received within the interior volume of the wearable computing device 100. Use of the light emitter(s) 129 may particularly be useful in dark environments, where there is little ambient light. Moreover, the light emitter(s) 129 may be controlled and/or configured to output specific wavelengths of light, infrared, ultraviolet, and/or the like, which may be used to generate particular biometric data. The light data generated by the light detector(s) 130 may be used to determine an optical measurement of a biometric, such as a heart rate (HR) measurement, blood oxygen saturation (SpO2) measurement, heart rate variability measurement, blood pressure, a SAF measurement, and/or other physiological metrics, and/or a distance of the rear housing cover 114 of the wearable computing device 100 from an object, such as the user's forearm 104 (FIG. 1A).

Moreover, as shown in the schematic diagram of a computing system 150 including the wearable computing device 100 in FIG. 2, the wearable computing device 100 may also include one or more internal motion sensors 138 (e.g., accelerometers, gyroscopes, and/or the like) within the interior volume of the housing 102 and configured to generate motion data indicative of movement of the wearable computing device 100.

In addition, as shown, the wearable computing device 100 includes any suitable user interface elements, such as the display 116 of the wearable computing device 100 and/or one or more additional I/O devices 140 configured to allow the wearable computing device 100 to receive conventional inputs from a user. These conventional inputs can include, for example, a push button (e.g., button 142 in FIG. 1B), wheel, joystick, keyboard, mouse, keypad, and/or any other such device or element whereby a user can input a command to the wearable computing device 100. In some embodiments, the I/O device(s) 140 may additionally, or alternatively, include a microphone or other audio capture element that accepts voice or other audio commands. For example, in particular embodiments, the wearable computing device 100 may be controllable through a combination of visual and audio commands, such that a user can control the wearable computing device 100 without having to specifically contact an I/O element of the wearable computing device 100. In certain embodiments, the I/O elements 140 may include the light emitter(s) 129, the light detector(s) 130, the temperature sensor(s), SAF sensor, humidity sensor, pressure sensor, microphone(s), biometric sensor(s), motion sensors, and/or any other sensor described herein.

Furthermore, as shown in FIG. 2, the wearable computing device 100 may include a temperature sensor 123 for generating skin temperature data of a user of the wearable computing device 100. In an embodiment, for example, the temperature sensor 123 may be a skin temperature sensor in contact with the user's skin. In addition, as shown, the wearable computing device 100 may include a SAF sensor 125 for generating SAF data of the user.

As further shown in FIG. 2, the wearable computing device 100 may also include at least one controller 144. In an embodiment, the controller(s) 144 may be a central processing unit (CPU) or graphics processing unit (GPU) for executing instructions that can be stored in a memory device 146, such as flash memory or DRAM, among other such options. For example, in an embodiment, the memory device 146 may include RAM, ROM, FLASH memory, or other non-transitory digital data storage, and may include a control program comprising sequences of instructions which, when loaded from the memory device 146 and executed using the controller(s) 144, cause the controller(s) 144 to perform the functions that are described herein. As would be apparent to one of ordinary skill in the art, the computing system 150 can include many types of memory, data storage, or computer-readable media, such as data storage for program instructions for execution by the controller or any suitable processor. The same or separate storage can be used for images or data, a removable memory can be available for sharing information with other devices, and any number of communication approaches can be available for sharing with other devices.

The wearable computing device 100 also includes one or more power components 147, such as a battery operable to be recharged, for powering one or more components of the wearable computing device 100 described above. The power component(s) 147 may be at least partially positioned within the interior volume of the wearable computing device 100.

The wearable computing device 100 may also include one or more wireless components 148 operable to allow the controller(s) 144 to communicate with one or more electronic devices within a communication range of the particular wireless channel. The wireless channel can be any appropriate channel used to enable devices to communicate wirelessly, such as Bluetooth, cellular, NFC, Ultra-Wideband (UWB), or Wi-Fi channels. It should be understood that the wearable computing device 100 can have one or more conventional wired communications connections as known in the art.

The user might have a wearable computing device, such as a smartwatch or fitness tracker (e.g., the wearable computing device 100), which the user would like to be able to communicate with other devices, such as a smartphone, a tablet computer, and/or the like. Applications may allow communication between multiple devices and a wearable computing device to enable a user to obtain information from the wearable computing device. For example, data captured using a sensor of the wearable computing device 100 may be communicated to the smartphone and/or the tablet computer using an application installed on the smartphone and/or the tablet computer. The user may also want the wearable computing device 100 to be able to communicate with a service provider, such as with the host computer 152 of a service provider, or other such entity, that is able to obtain and process data from the wearable computing device 100 and provide functionality that may not otherwise be available on the wearable computing device 100 or applications installed on the other devices. In some embodiments, the host computer 152 executes control programs and/or application programs that are configured to perform some of the functions described herein.

In addition, as shown, the wearable computing device 100 may be able to communicate with the service provider (e.g., host computer 152 of the service provider) through at least one network (e.g., network 154), such as the Internet or a cellular network, one or more local area networks, wide area networks, UWB, and/or internetworks using any of terrestrial or satellite links, and/or may communicate over a wireless connection such as Bluetooth^{®} to the other device(s) (e.g., the smartphone and/or the tablet computer), where the other device(s) then communicate with the service provider over the at least one network. There may be a number of other types of, or reasons for, communications in various embodiments.

In addition to being able to communicate, a user may also want the devices to be able to communicate in a number of ways or with certain aspects. For example, the user may want communications between the devices to be secure, particularly where the data may include personal health data or other such communications. The device or application providers may also be required to secure this information in at least some situations. The user may want the devices to be able to communicate with each other concurrently, rather than sequentially. This may be particularly true where pairing may be required, as the user may prefer that each device be paired at most once, such that no manual pairing is required. The user may also desire the communications to be as standards-based as possible, not only so that little manual intervention is required on the part of the user but also so that the devices can communicate with as many other types of devices as possible, which is often not the case for various proprietary formats. A user may thus desire to be able to walk in a room with one device and have such device automatically communicate with another target device with little to no effort on the part of the user. In various conventional approaches, a device will utilize a communication technology such as Wi-Fi to communicate with other devices using wireless local area networking (WLAN). Smaller or lower capacity devices, such as many Internet of Things (IoT) devices, instead utilize a communication technology such as Bluetooth^{®}, and in particular Bluetooth Low Energy (BLE) which has very low power consumption.

Further to the descriptions above, a user may be provided with privacy-related controls allowing the user to make an election as to both if and when systems, programs, or features described herein may enable collection of health-related data and/or user information (e.g., information about a user's social network, social actions, or activities, profession, a user's preferences, or a user's current location), and if the user is sent content or communications that may be of a sensitive or private nature from a server. In addition, certain data may be treated in one or more ways before it is stored or used, so that personally identifiable information is removed. For example, a user's identity may be treated so that no personally identifiable information can be determined for the user, or a user's geographic location may be generalized where location information is obtained (such as to a city, ZIP code, or state level), so that a particular location of a user cannot be determined. Thus, the user may have control over what information is collected about the user, how that information is used, and what information is provided to the user. To that end, any information collected as described herein relating to the user (e.g., personal medical data, health conditions, etc.) is capable of being kept private and confidential and not being improperly used or published.

In further embodiments, data may be captured, processed, and displayed in a number of different ways. For example, data may be captured using sensors on the wearable computing device 100, but due to limited resources on the wearable computing device 100, the data may be transferred to the smartphone, the tablet computer, and/or the service provider (or a cloud resource) for processing, and results of that processing may then be presented back to that user on the wearable computing device 100, smartphone, the tablet computer and/or another such device associated with that user. In at least some embodiments, a user may also be able to provide input such as health data using an interface on any of these devices, which can then be considered when making that determination.

Skin autofluorescence (SAF) is a valuable biomarker of cardiovascular risk. Maximizing the value of SAF requires monitoring small changes in SAF over time. Unfortunately, as mentioned, SAF is sensitive to skin temperature, with higher temperatures resulting in lower SAF and vice versa. The magnitude of the SAF change with moderate changes in skin temperature is large relative to the change in SAF expected from behavior change over a few months. This means that it can be difficult to interpret changes in SAF correctly. This temperature sensitivity has been under-appreciated in the industry, especially in the use of SAF as a biomarker of cardiovascular risk, resulting in some incorrect interpretations of results. As a consequence, existing technologies for measuring SAF do not consider temperature.

Accordingly, the present disclosure is directed to the wearable computing device 100 described herein, having both the temperature sensor 123 and the SAF sensor 125 in the same device. Thus, the controller(s) 144 described herein is configured to correct for skin temperature when evaluating changes in SAF signals over time. Moreover, in an embodiment, the temperature sensor 123 is configured to generate skin temperature data of a user of the wearable computing device 100. Further, in an embodiment, the SAF sensor 125 is configured to generate SAF data of the user.

As such, the controller(s) 144 described herein is configured to perform a plurality of operations, such as receiving the skin temperature data and the SAF data. In further embodiments, the controller(s) 144 is configured to classify the skin temperature data into different groups of temperature data based on different causes of the fluctuations in the skin temperature of the user having different origins. For example, in such embodiments, the different causes of the fluctuations in the skin temperature of the user having different origins may include weather, season, environment, exercise, heredity, age, gender, fitness level, location, illness, stress, or similar.

Further, in an embodiment, the controller(s) 144 may optionally determine a correlation between the skin temperature data and the SAF data over time. For example, in an embodiment, the controller(s) 144 may determine the correlation between the skin temperature data and the SAF data over time using linear regression and/or a machine-learned model. This may also be completed by a separate controller apart from the wearable computing device 100. In such embodiments, the controller(s) 144 can apply predetermined temperature correction factors to the measurements of SAF and skin temperature data over time.

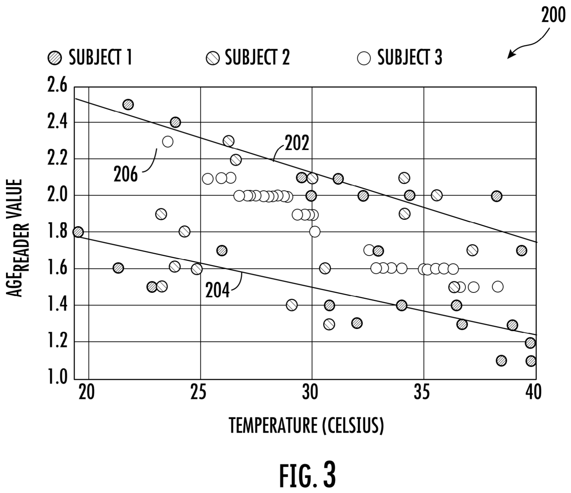

As an example, FIG. 3 illustrates a graph 200 of advanced glycation end products (AGEs) data versus the skin temperature data over time in accordance with aspects of the present disclosure. In particular, as shown, three different subjects (e.g., Subject 1, Subject 2, Subject 3) are plotted to illustrate correlations 202, 204, 206 between AGEs data and the skin temperature data over time, e.g., using linear regression. Thus, as shown, the correlations 202, 204, 206 show that, as skin temperature increases (x-axis), SAF decreases and vice versa. However, as shown, the correlations 202, 204, 206 vary from subject to subject. Thus, as shown, by combining the temperature sensor 123 and the SAF sensor 125 together in one device and monitoring how these two parameters vary together, a personalized temperature correction can be developed.

Accordingly, the controller(s) 144 described herein is configured to determine and/or apply a temperature correction factor based on the correlation. For example, in an embodiment, the temperature correction factor may be predetermined using one or more experiments, e.g., relating to the relationship between the skin temperature data and the SAF data. Thus, in an embodiment, the controller(s) 144 is configured to apply the temperature correction factor to the SAF data to correct the SAF data for skin temperature of the user. In another embodiment, the controller(s) 144 may be preprogrammed with the temperature correction factor such that the controller(s) 144 can omit the determining step and simply apply the correction factor to the SAF data.

In such embodiments, the controller(s) 144 may include one or more algorithms for correcting for the temperature. In particular embodiments, the method of obtaining this correction may be accomplished through a regression analysis of controlled experiments. A personalized correction might be desirable as the magnitude of the skin temperature sensitivity can vary across people.

In an embodiment, for example, the controller(s) 144 described herein is configured to apply the temperature correction factor to the SAF data to correct the SAF data for the skin temperature of the user when the skin temperature data varies over a large enough range in a short enough time period. However, for instances in which a user's natural skin temperature does not vary over a large enough range in a short enough time to produce a reliable correction, the wearable computing device 100 can be heated using one or more heating elements, such as one or more extant components of the wearable computing device 100 (e.g. the display 116 and/or cellular connection components) to create a large enough skin temperature range. Thus, in such embodiments, the controller(s) 144 described herein is configured to opportunistically measure SAF at extreme outliers in temperature (such measurements of skin temperature of at least 40°C, less than about 15°C for about 10 minutes or more, etc.), which enables further improvement of the temperature correction algorithm, as well as individual corrections.

In addition, the controller(s) 144 described herein is configured to determine at least one health parameter of the user using the corrected SAF data and displaying, via the display 116 of the wearable computing device 100, the health parameter(s) to the user. For example, in an embodiment, the health parameter may be cardiovascular health of the user.

It should be appreciated that the algorithm(s) described herein can be or include one or multiple machine-learned models or model components. Example machine-learned models can include neural networks (e.g., deep neural networks). Example machine-learned models can include non-linear models or linear models. Example machine-learned models can use other architectures in lieu of or in addition to neural networks. Example machine-learned models can include decision tree based models, support vector machines, hidden Markov models, Bayesian networks, linear regression models, k-means clustering models, etc. In some instances, the models may be trained on training information for known/measured contact between the user and the sensor(s) described herein, known/measured biometrics of the user during such feedback using sensors other than those of the wearable computing device 100, and the output of the sensor(s) of the wearable computing device 100 during such known conditions.

Referring now to FIG. 3, a flow diagram of one embodiment of a method 300 of correcting skin autofluorescence (SAF) data based on skin temperature of a user is provided. In an embodiment, for example, the wearable computing device may be any suitable wearable computing device, such as the wearable computing device 100 described herein with reference to FIGS. 1A-3. Thus, in general, the method 300 is described herein with reference to the wearable computing device 100 of FIGS. 1A-2, and the computing system 150 described in FIG. 2 including the wearable computing device 100. However, it should be appreciated that the disclosed method 300 may be implemented with any other suitable wearable computing device having any other suitable configurations and/or with any other suitable system. In addition, although FIG. 3 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

As shown at (302), the method 300 may include receiving, via a computing device, skin temperature data and the SAF data of the user over time. As shown at (304), the method 300 may include determining, via the computing device, a correlation between the skin temperature data and the SAF data over time. As shown at (306), the method 300 may include applying, via the computing device, a temperature correction factor to the SAF data to correct the SAF data based on the skin temperature of the user based on the correlation. As shown at (308), the method 300 may include determining, via the computing device, at least one health parameter of the user using the corrected SAF data. As shown at (310), the method 300 may include displaying, via a display of the computing device, the health parameter(s) to the user.

The technology discussed herein makes reference to servers, databases, software applications, and other computer-based systems, as well as actions taken and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

While the present disclosure has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present disclosure as would be readily apparent to one of ordinary skill in the art. For instance, features illustrated or described as part of one embodiment can be used with another embodiment to yield a still further embodiment. Thus, it is intended that the present disclosure covers such alterations, variations, and equivalents.

## Claims

1. A computing device, comprising:
a temperature sensor for generating skin temperature data of a user of the computing device;
a skin autofluorescence, SAF, sensor for generating SAF data of the user; and
a controller configured to perform a plurality of operations, the plurality of operations comprising:
receiving the skin temperature data and the SAF data over time;
determining a correlation between the skin temperature data and the SAF data over time;
applying a temperature correction factor to the SAF data to correct the SAF data for skin temperature of the user based on the correlation;
determining at least one health parameter of the user using the corrected SAF data; and
displaying, via a display of the computing device, the at least one health parameter to the user.

2. The computing device of claim 1, wherein determining the correlation between the skin temperature data and the SAF data over time further comprises generating a graph of advanced glycation end products, AGEs, data versus the skin temperature data over time and correlating the AGEs data and the skin temperature data using linear regression.

3. The computing device of claim 1 or 2, wherein the plurality of operations further comprises applying the temperature correction factor to the SAF data to correct the SAF data for the skin temperature of the user when the skin temperature data varies from the SAF data over a large enough range in a short enough time period.

4. The computing device of claim 3, further comprising one or more heating elements, the one or more heating elements for heating one or more components of the computing device when the correlation indicates that the skin temperature data of the user does not vary from the SAF data over the large enough range in the short enough time period.

5. The computing device of claim 4, wherein the one or more heating elements comprise at least one of a display or a cellular connection component of the computing device.

6. The computing device of any one of the preceding claims, wherein the plurality of operations further comprises classifying the skin temperature data into different groups of temperature data based on different causes of fluctuations in the skin temperature of the user having different origins.

7. The computing device of claim 6, wherein the different causes of the fluctuations in the skin temperature of the user having different origins comprise at least one of weather, season, environment, exercise, heredity, age, gender, fitness level, location, illness, or stress.

8. The computing device of any one of the preceding claims, wherein the at least one health parameter is indicative of cardiovascular health of the user.

9. The computing device of any one of the preceding claims, wherein the temperature sensor is in contact with the user's skin.

10. The computing device of any one of the preceding claims, wherein the computing device is a wearable computing device.

11. A method of correcting skin autofluorescence, SAF, data based on skin temperature of a user, the method comprising:
receiving, via a computing device, skin temperature data and the SAF data of the user over time;
determining, via the computing device, a correlation between the skin temperature data and the SAF data over time;
applying, via the computing device, a temperature correction factor to the SAF data to correct the SAF data based on the skin temperature of the user based on the correlation;
determining, via the computing device, at least one health parameter of the user using the corrected SAF data; and
displaying, via a display of the computing device, the at least one health parameter to the user.

12. The method of claim 11, wherein determining the correlation between the skin temperature data and the SAF data over time further comprises generating a graph of advanced glycation end products, AGEs, data versus the skin temperature data over time and correlating the AGEs data and the skin temperature data using linear regression.

13. The method of claim 11 or 12, further comprising applying the temperature correction factor to the SAF data to correct the SAF data for the skin temperature of the user when the skin temperature data varies from the SAF data over a large enough range in a short enough time period.

14. The method of claim 13, further comprising one or more heating elements, the one or more heating elements for heating one or more components of the computing device when the correlation indicates that the skin temperature data of the user does not vary from the SAF data over the large enough range in the short enough time period.

15. The method of claim 14, wherein the one or more heating elements comprise at least one of a display or a cellular connection component of the computing device.
